# EUROPÄISCHE PATENTANMELDUNG

(11) **EP 2 357 037 A1**
(43) Veröffentlichungstag der Anmeldung: **17.08.2011**
(21) Anmeldenummer: 10153864.3
(22) Anmeldetag: 17.02.2010
(51) Int. Cl.: B01J 23/80, B01J 37/00, B01J 37/03, B01J 37/18, C07C 29/149, C07C 31/20

(54) **Verfahren zur Herstellung von mechanisch stabilen Katalysatorformkörpern**

(71) Anmelder: LANXESS Deutschland GmbH, 51369 Leverkusen (DE)
(72) Erfinder: Boll, Matthias, 51061, Köln (DE); Heuer, Lutz, 41542, Dormagen (DE)

(57) **Zusammenfassung**

Die vorliegende Erfindung betrifft Katalysatorformkörper enthaltend Kupfer, Zink und Aluminium, ein Verfahren zu ihrer Herstellung sowie die Verwendung der erfindungsgemäßen Katalysatorformkörper in einem Verfahren zur Herstellung von Alkoholen durch Umsetzung von Carbonsäuren und/oder Carbonsäureestern mit Wasserstoff.

## Beschreibung

Die vorliegende Erfindung betrifft Katalysatorformkörper enthaltend Kupfer, Zink und Aluminium, ein Verfahren zu ihrer Herstellung sowie die Verwendung der erfindungsgemäßen Katalysatorformkörper in einem Verfahren zur Herstellung von Alkoholen durch Umsetzung von Carbonsäuren und/oder Carbonsäureestern mit Wasserstoff.

Die katalytische Hydrierung von Carbonylverbindungen wie beispielsweise Carbonsäuren oder Carbonsäureestern hat in der chemischen Grundstoffindustrie eine bedeutende Stellung erlangt.

Etablierte technische Verfahren werden dabei fast ausschließlich in Festbettreaktoren durchgeführt, wobei als Festbettkatalysatoren neben Katalysatoren vom Raney-Typ insbesondere geträgerte Katalysatoren beispielsweise auf Kupfer-, Nickel- oder Edelmetall-Basis verwendet werden.

Aufgrund der Verwendung in einem Festbett ist es von entscheidender Bedeutung, dass der Katalysator während der Reaktion formstabil bleibt, da ansonsten bei der Bildung von Bruchstücken oder pulverförmigem Abrieb eine unerwünschte Druckerhöhung im Festbett auftreten kann und Katalysator teilweise aus dem Festbett ausgetragen wird. Ausgetragener Katalysator kann sich in anderen Anlagenteilen ablagern und dort erhebliche Störungen verursachen.

Die Hydrierung von Carbonsäuren und Carbonsäureestern in Gegenwart von Katalysatoren auf Basis von Kupferchromit, dem sogenannten Adkins-Katalysator ist seit Langem bekannt. Die Verwendung von chromhaltigen Katalysatoren ist jedoch aus Gründen der damit verbundenen Gefahren für die Umwelt nicht wünschenswert. Es sind daher Anstrengungen unternommen worden, diese chromhaltigen Katalysatoren durch umweltfreundlichere, chromfreie Katalysatoren zu ersetzen.

So ist aus WO 82/03854 A1 ein Verfahren zur Hydrierung von Carbonsäureestern bekannt, in dem ein Katalysators eingesetzt wird, der eine reduzierte Mischung aus Kupferoxid und Zinkoxid enthält.

In US 3,923,694 sind Katalysatoren enthaltend Kupfer, Zink und Aluminium beschrieben, die jedoch während der Reaktion mechanisch nicht ausreichend stabil sind und daher relativ schnell zerfallen.

In DE 198 09 418 A ist ein Verfahren zur katalytischen Hydrierung einer Carbonylverbindung in Gegenwart eines Katalysators beschrieben, der einen Träger, insbesondere Titandioxid enthält, und als Aktivkomponente Kupfer oder ein Gemisch aus Kupfer mit mindestens einem weiteren Metall umfasst, wobei das weitere Metall ausgewählt ist aus der Gruppe Zink, Aluminium, Cer, einem Edelmetall und einem Metall der VIII. Nebengruppe. Die spezifische Oberfläche beträgt dabei maximal 10 m²/g.

EP 0 721 928 A beschreibt ein Verfahren zur Herstellung von aliphatischen α,ω-Diolen durch Hydrierung der entsprechenden Carbonsäureester mit einem Katalysator, der eine reduzierte Mischung aus verpressten Pulvern von Kupfer-, Zink- und Aluminiumoxiden enthält, wobei der Katalysator weiterhin Eisen-, Nickel- oder Manganoxid enthalten kann.

US 5 155 086 beschreibt pulverförmige Hydrierkatalysatoren auf Basis von Kupfer, Zink und Aluminium, die eine Hauptmenge an Oxiden des Kupfers und Zinks und geringere Mengen an Aluminiumoxid enthalten, wobei das Porenvolumen der Poren, die einen Durchmesser zwischen 120 und 1000 Å aufweisen, mindestens 40 % des gesamten Porenvolumens beträgt. Die Katalysatoren sollen sich insbesondere zur Hydrierung von Aldehyden, Ketonen, Carbonsäuren und Carbonsäureestern eignen.

Katalysatoren auf Basis von Kupfer, Zink und Aluminium werden häufig in der Methanolsynthese eingesetzt (US 4 305 842, EP 0 125 689 A). Aluminiumoxid und Zinkoxid haben bei diesen Katalysatoren die Funktion eines Trägermaterials für Kupfer. Derartige Katalysatoren werden beispielsweise durch Co-Fällung der Komponenten hergestellt und durch Kalzinierung und Reduktion zu aktiven Katalysatoren umgewandelt.

So sind aus EP 0 125 689 A Hydrierkatalysatoren auf Basis von Kupfer, Zink und Aluminium bekannt, bei deren Herstellung kolloidales Titandioxid oder Aluminiumhydroxide zum Produkt der Co-Fällung von Kupfer und Zink zugesetzt werden.

JP 09-173845 A beschreibt die Herstellung von Kupfer und Zink enthaltenden Katalysatoren, die durch die Tränkung von γ-Aluminiumoxiden mit wasserlöslichen Zink- und Kupfer-Salzen hergestellt werden, sowie die Verwendung dieser Katalysatoren in der Synthese von Dimethylether.

WO 99/4974 A beschreibt Katalysatoren, die durch die Auffällung von Kupfer und Zink auf Titandioxid hergestellt werden. Zur Herstellung von Tabletten aus dem pulverförmigen Katalysator wird als Tablettierhilfsmittel metallisches Kupferpulver zugesetzt, um eine ausreichende Härte zu erzielen.

In DE 199 42 895 A und DD 256 515 ist ebenfalls die Wirkung von metallischem Kupfer oder Zement als Tablettierhilfsmittel zur Herstellung von Kupfer, Zink und Aluminium enthaltenden Katalysatoren zur Hydrierung von Carbonsäureestern beschrieben. Auch hier führt dieser Zusatz zu einer Erhöhung der Seitendruckfestigkeit.

WO 97/34694 A beschreibt Kupfer, Zink und Aluminium enthaltende Katalysatoren mit einem Aluminiumgehalt von über 20 %, die in Form von Extrudaten eine bimodale Porengrößenverteilung aufweisen. Diese Katalysatoren sollen für die Hydrierung von Fettsäureestern gut geeignet sein.

Schließlich offenbart DE 102 07 443 A ein Verfahren zur Herstellung von Alkoholen durch Umsetzung von Carbonsäuren oder Carbonsäureestern mit Wasserstoff in Gegenwart eines Katalysators, der in nicht reduziertem Zustand 20 bis 80 Gew.-% Kupferoxid, 10 bis 80 Gew.-% **Zinkoxid und 1 bis** 50 Gew.-% Aluminiumoxid enthält und eine Porengrößenverteilung aufweist, bei der 5 bis 15 % des Gesamtporenvolumens im Porendurchmesserbereich unterhalb von 15 nm und 80 bis 95 % des Gesamtporenvolumens im Porendurchmesserbereich oberhalb von 25 nm liegen.

Den im Stand der Technik beschriebenen Katalysatoren ist gemeinsam, dass sie unter typischen Hydrierbedingungen und den dabei auftretenden mechanischen Belastungen nur begrenzte mechanische Stabilität aufweisen, was aufgrund der daraus resultierenden häufigeren Betriebsunterbrechungen für die Erreichung hoher Raum-Zeit-Ausbeuten kontraproduktiv ist.

Aufgabe der Erfindung war es daher einen Katalysator und ein Verfahren zu seiner Herstellung bereitzustellen, der unter typischen Hydrierbedingungen eine hohe mechanische Stabilität bei gleichzeitig hoher Aktivität aufweist.

Es wurde nun ein Verfahren zur Herstellung eines Kupfer, Zink und Aluminium enthaltenden Katalysators gefunden, das dadurch gekennzeichnet ist, dass es zumindest folgende Schritte umfasst:
A) Herstellung eines Katalysators durch
   - Co-Fällung zumindest einer im Wesentlichen wasserlöslichen Kupferverbindung, zumindest einer im Wesentlichen wasserlöslichen Zinkverbindung und zumindest einer im Wesentlichen wasserlöslichen Aluminiumverbindung und/oder
   - Aufflällung zumindest einer im Wesentlichen wasserlöslichen Kupferverbindung und zumindest einer im Wesentlichen wasserlöslichen Zinkverbindung auf zumindest ein Aluminium und Sauerstoff enthaltendes Trägermaterial
   in einem wässrigen Reaktionsmedium
B) Abtrennung des gemäß Schritt A) erhaltenen, gefällten Katalysators vom wässrigen Reaktionsmedium
C) Herstellung von Katalysatorformkörpern enthaltend den gemäß Schritt B) erhaltenen, gefällten Katalysator
wobei das Verfahren weiterhin dadurch gekennzeichnet ist, dass es keine Kalzinierung des gefällten Katalysators oder der Katalysatorformkörper umfasst.

Der Rahmen der Erfindung umfasst die vor- und nachstehend aufgeführten, allgemeinen oder in Vorzugsbereichen aufgeführten Restedefinitionen bzw. Parameter auch in beliebigen Kombination untereinander.

Der Begriff "wässriges Reaktionsmedium" bedeutet im Rahmen der Erfindung ein flüssiges, Wasser enthaltendes Medium, das vorzugsweise mindestens 50 Gew.- % Wasser enthält und besonders bevorzugt frei von organischen Lösungsmitteln ist.

Der Begriff "reduziert" oder "reduzierter Zustand" bedeutet im Rahmen der Erfindung bezogen auf Katalysatoren, dass mindestens 90%, vorzugsweise mindestens 95 % und besonders bevorzugt mindestens 98 % des im Katalysator enthaltenen Kupfers in elementarer Form vorliegen.

Dementsprechend bedeutet der Begriff "nicht-reduziert" oder "nicht-reduzierter Zustand" bezogen auf Katalysatoren, dass mindestens 90%, vorzugsweise mindestens 95 % und besonders bevorzugt mindestens 98 % des im Katalysator enthaltenen Kupfers nicht in elementarer Form vorliegt.

Im Rahmen der Erfindung ist unter dem Begriff "Kalzinierung" eine thermische Behandlung des Katalysators oder der Katalysatorformkörper unter nicht-reduzierenden Bedingungen zu verstehen, bei der der anschließend beobachtete Gewichtsverlust einer Probe des kalzinierten Katalysators oder der kalzinierten Katalysatorformkörper beim 4 stündigen Erhitzen auf 600°C gegenüber einer Vergleichsprobe des nicht-kalzinierten Katalysators oder der nicht-kalzinierten Katalysatorformkörper, die jedoch bei 110°C über 12 Stunden getrocknet wurden, relativ gesehen mindestens 10 % geringer ist.

Zeigt beispielsweise eine Katalysatorprobe, die bei 110°C über 12 Stunden getrocknet wurde, beim 4-stündigen Erhitzen auf 600°C einen Gewichtsverlust von 22,0 %, so ist unter einer Kalzinierung jede thermische Behandlung zu verstehen, die zu einer Modifikation des Katalysators derart führt, dass der Gewichtsverlust beim 4-stündigen Erhitzen auf 600°C weniger als 19,8 Gew.-% beträgt.

Der Gewichtsverlust beim Kalzinieren ist typischerweise auf Eliminierungsreaktionen von Wasser aus Hydroxiden oder Kohlendioxid aus Carbonaten zurückzuführen

Typische Kalzinierbedingungen umfassen dabei Erhitzen des Katalysators oder der Katalysatorformkörper unter nicht-reduzierenden Bedingungen auf 300°C oder mehr, vorzugsweise 300 bis 1000°C, besonders bevorzugt 300 bis 700°C, wobei das Erhitzen typischerweise für 5 min bis 24 Stunden, vorzugsweise für 1 bis 24 Stunden erfolgt.

Nicht-reduzierende Bedingungen sind solche Bedingungen, die nicht geeignet sind, den Katalysator in den reduzierten Zustand gemäß obenstehender Definition zu versetzen.

Gemäß Schritt A) wird ein Katalysators durch Auf- oder Co-Fällung von Metallverbindungen auf oder mit einem Aluminium und Sauerstoff enthaltenden Trägermaterial in einem wässrigen Reaktionsmedium hergestellt, wobei als Metallverbindungen zumindest eine Kupferverbindung und zumindest eine Zinkverbindung eingesetzt werden.

Der Begriff Auffällung in einem wässrigen Reaktionsmedium umfasst die Konvertierung von im Wesentlichen wasserlöslichen Kupfer- und Zinkverbindungen in im Wesentlichen wasserunlösliche Kupfer- und Zinkverbindungen auf im Wesentlichen wasserunlösliche Trägermaterialien enthaltend Aluminium und Sauerstoff, die zur Konvertierung in das wässrige Reaktionsmedium eingebracht wurden.

Der Begriff Co-Fällung in einem wässrigen Reaktionsmedium umfasst die Konvertierung von in Wesentlichen wasserlöslichen Kupfer- und Zinkverbindungen und im Wesentlichen wasserlöslichen Aluminiumverbindungen in im Wesentlichen wasserunlösliche Kupfer- und Zinkverbindungen und im Wesentlichen wasserunlösliche Trägermaterialien enthaltend Aluminium und Sauerstoff.

Im Wesentlichen wasserlösliche Kupfer-, Zink- und Aluminiumverbindungen sind solche, die bei 20°C und einem Druck von 1013 hPa eine Wasserlöslichkeit von mindestens 5,0 g/l aufweisen.

Im Wesentlichen wasserunlösliche Kupfer-, Zink- und Aluminiumverbindungen sind dementsprechend solche, die bei 20°C und einem Druck von 1013 hPa eine Wasserlöslichkeit von weniger als 5,0 g/l aufweisen.

Geeignete, im Wesentlichen wasserlösliche Kupferverbindungen sind beispielsweise Kupfer(II)-nitrat, Kupfer(II)sulfat, Kupfer(II)chlorid, Kupfer(II)bromid, Kupfer(I)acetat, Kupfer(II)acetat oder deren Hydrate oder Mischungen der vorgenannten Verbindungen, wobei Kupfer(II)sulfat und Kupfer(II)nitrat bzw. deren Hydrate bevorzugt sind.

Geeignete, im Wesentlichen wasserlösliche Zinkverbindungen sind beispielsweise Zinknitrat, Zinksulfat, Zinkchlorid, Zinkbromid, Zinkacetat, Zinkcitrat oder deren Hydrate oder Mischungen der vorgenannten Verbindungen, wobei Zinknitrat und Zinksulfat bzw. deren Hydrate bevorzugt sind.

Für Co-Fällungen geeignete, im Wesentlichen wasserlösliche Aluminiumverbindungen sind beispielsweise Aluminiumnitrat, Aluminiumsulfat, Aluminiumacetat, Aluminiumchlorid Aluminiumcitrat, sowie gegebenenfalls deren Hydrate und deren Mischungen, wobei Aluminiumnitrat und Aluminiumsulfat bevorzugt sind.

Für Auffällungen verwendete Aluminium und Sauerstoff enthaltende Trägermaterialien können beispielsweise sein:
Verbindungen, die mehr als 90 Gew.-%, bevorzugt mehr als 95 Gew.% an Aluminium und Sauerstoff enthalten wie beispielsweise Aluminiumoxide wie insbesondere γ-Aluminiumoxide, α-Aluminiurnoxide, β-Aluminiumoxide, Böhmit, Tonerden, Kaolin, Bauxit, Gibbsite, Aluminiumoxidhydroxide, Aluminiumhydroxide wechselnder Zusammensetzung und mit wechselndem Wassergehalt, Aluminiumoxide natürlicher Herkunft oder synthetische Aluminiumoxide.

Bevorzugt werden Trägermaterialien mit einer Partikelgröße (zahlengemittelter Durchmesser) von 1 bis 100 µm, besonders bevorzugt 3 bis 80 µm, insbesondere bevorzugt 10 bis 50 µm eingesetzt.

Vorzugsweise werden pulverförmige Trägermaterialien mit einer spezifischen Oberfläche (bestimmt nach DIN 66133) von 0,1 bis 400 m²/g, besonders bevorzugt 100 bis 300 m²/g eingesetzt.

Vorzugsweise werden pulverförmige Trägermaterialien mit einem Porenvolumen von 0,1 bis 1,5 ml/g, besonders bevorzugt 0,4 bis 0,8 ml/g eingesetzt. Sofern nicht anders angegeben wurden Porenvolumina an einem Poremaster 60-GT der Quantachrome gemessen.

Vorzugsweise werden pulverförmige Trägermaterialien mit einem Natriumgehalt von weniger als 2 Gew.-%, bevorzugt 0,01 bis 0,1 Gew.-% eingesetzt.

Vorgenannte Aluminium und Sauerstoff enthaltende Trägermaterialien können natürlich auch durch Co-Fällung erzeugt werden.

In einer bevorzugten Ausführungsform erfolgt die Herstellung des Katalysators im Schritt A) jedoch durch Auffällung.

Auf- und Co-Fällung bzw. die oben genannte Konvertierung erfolgen vorzugsweise derart, dass der pH-Wert des wässrigen Reaktionsmediums bei der Konvertierungstemperatur durch Zugabe von Basen auf 6,0 bis 12,0; vorzugsweise 7,5 bis 9,0 und besonders bevorzugt 7,7 bis 8,5 eingestellt wird.

Die Temperatur, bei der die Auffällung oder Co-Fällung erfolgt (Konvertierungstemperatur) beträgt beispielsweise 0 bis 100°C, vorzugsweise 20 bis 85°C und besonders bevorzugt 50 bis 80°C.

Geeignete Basen sind vorzugsweise solche, die in Wasser einen pK_{B}-Wert bei Standardbedingungen von 5,0 oder weniger, vorzugsweise 4,0 oder weniger aufweisen.

Beispielhaft seien Alkalimetallcarbonate und -hydroxide wie beispielsweise Lithium-, Natrium- oder Kaliumcarbonat sowie Lithium-, Natrium oder Kaliumhydroxid genannt, wobei die Verwendung von Alkalimetallcarbonaten wie insbesondere Natrium- oder Kaliumcarbonat oder Mischungen davon noch weiter bevorzugt ist.

Bei der Verwendung von Carbonaten wird bei der Fällung typischerweise die Bildung von Malachit, einem monoklinen basischen Kupfercarbonat mit der Zusammensetzung Cu₂[(OH)₂|CO₃] als kristalliner Bestandteil beobachtet. Vorzugsweise wird die Wahl der Temperatur- und pH-Bedingungen bei der Auf- oder Co-Fällung so gewählt, dass zumindest 30 Mol-%, bevorzugt zumindest 70 Mol-% der kristallin gefällten kupferhaltigen Verbindung in Form von Malachit mit einer durchschnittlichen Kristallitgröße von 1 bis 80 nm, vorzugsweise 5 bis 60 nm vorliegt. Geeignete Bedingungen sind durch wenige Vorversuche leicht ermittelbar und das Ergebnis durch Röntgenstrukturanalyse z.B. Anhand Berechnung mit Hilfe des Form des Reflexes [0/2/0] leicht überprüfbar (Kristallitgrößenabschätzung nach der Scherrer-Gleichung). Sofern nicht anders angegeben, erfolgte die Messung der Kristallitgröße jeweils auf einem Reflexions-Diffraktometer Siemens XRD D 5000 mit automatischer Aperturblende.

Vorzugsweise wird das Verhältnis von Kupfer-, Zink- und Aluminiumverbindungen bei der Co-Fällung bzw. das Verhältnis von Kupfer- und Zinkverbindungen- und Aluminium und Sauerstoff enthaltenden Trägermaterialien sowie die Reaktionsbedingungen so gewählt, dass der schließlich gemäß Schritt B) erhaltene Katalysator in nicht-reduziertem Zustand bezogen auf sein Trockengewicht 2 bis 60 Gew.-% Kupfer (bezogen auf das Metall), 2 bis 25 Gew.-% an Zink (bezogen auf das Metall) und 0,1 bis 10 Gew.-% an Aluminium (bezogen auf das Metall) enthält.

Weiterhin bevorzugt ist ein Gewichtsverhältnis von Kupfer und Zink von 1:1 1 bis 10:1 bezogen auf die Metalle, bevorzugt 2:1 bis 4:1.

Ebenfalls bevorzugt ist ein Gewichtsverhältnis von Kupfer und Aluminium von 10:1 bis 50:1 bezogen auf die Metalle , bevorzugt 20:1 bis 40:1.

Geeignete Verhältnisse sind in einfacher Weise beispielsweise durch Berechnung oder durch wenige Vorversuche ermittelbar. Werden die Metallverbindungen im Wesentlichen vollständig auf- oder co-gefällt ist sogar eine einfache Berechnung ausreichend.

Besonders bevorzugt wird das Verhältnis von Metallverbindungen und Aluminium und Sauerstoff enthaltenden Trägermaterialien sowie die Reaktionsbedingungen so gewählt, dass der gemäß Schritt B) erhaltene Katalysator in nicht-reduziertem Zustand bezogen auf sein Trockengewicht 40 bis 60 Gew.-% Kupfer (bezogen auf das Metall), 8 bis 15 Gew.-% an Zink (bezogen auf das Metall) und 0,8 bis 2,5 Gew.-% an Aluminium (bezogen auf das Metall) enthält.

Die Katalysatoren können zusätzlich entweder keine, eine oder mehrere weitere Metallverbindungen enthalten. Solche weiteren Metallverbindungen sind beispielsweise Verbindungen der Seltenerdmetalle, Alkalimetalle und Erdalkalimetalle, sowie Verbindungen von Zirkon, Titan, Cobalt, Molybdän, Vanadium, Wolfram, Eisen, Nickel, Mangan oder Rhenium oder Mischungen der vorgenannten Metalle oder Verbindungen.

Solche weiteren Metallverbindungen können beispielsweise in einer Menge von 0,1 bis 3 Gew.-% bezogen auf das Metall im Katalysator bezogen auf das Trockengewicht enthalten sein.

Die Einbringung der weiteren Metallverbindungen in den Katalysator erfolgt beispielsweise durch Auf- oder Co-Fällung der Metallverbindungen analog zu den Kupfer- und Zinkverbindungen oder durch Zusatz oder einen vorhandenen Gehalt des Aluminium und Sauerstoff enthaltenden Trägermaterials an den entsprechenden Metallverbindungen.

Im Schritt B) erfolgt die Abtrennung des gemäß Schritt A) erhaltenen, gefällten Katalysators vom wässrigen Reaktionsmedium.

Die Abtrennung kann beispielsweise durch Filtration, Zentrifugation oder Sedimentation und Dekantieren oder andere dem Fachmann bekannten Fest-Flüssig-Trennoperationen erfolgen, wobei Filtration bevorzugt ist.

Dem Schritt B) kann sich optional, aber bevorzugt ein Schritt B2) anschließen, in dem der abgetrennte gefällte Katalysator gewaschen wird. Das Waschen erfolgt ein- oder mehrmals z.B. mit Wasser.

Dem Schritt B) oder B2) kann sich optional, aber bevorzugt ein Schritt B3) anschließen, in dem der abgetrennte gefällte Katalysator getrocknet wird. Das Trocknen erfolgt beispielsweise bei Umgebungsdruck oder unter vermindertem Druck und bei Temperaturen von beispielsweise 20 bis 300°C, bevorzugt 50 bis 200°C, besonders bevorzugt 90 bis 120°C und wird solange durchgeführt, dass eine Kalzinierung gemäß obiger Definition nicht stattfindet.

Den Schritten B), B2) oder B3) kann sich optional, aber bevorzugt ein Schritt B4) anschließen, in dem der abgetrennte, gefällte Katalysator gemahlen wird.

Die Mahlung des Katalysators kann in an sich bekannter Weise beispielsweise durch eine Kugelmühle oder einen Mixer erfolgen. Die Mahlung führt beispielsweise zu einem Schüttgewicht von 500 bis 1400 g/l, vorzugsweise von 700 bis 1100 g/l.

Gemäß Schritt B) bzw. den optionalen Schritten B2), B3) und B4) wird ein Katalysator in nicht-reduziertem Zustand erhalten.

Das Gesamtporenvolumen der gemäß Schritt B) bzw. den optionalen Schritten B2), B3) und B4) erhaltenen Katalysatoren liegt beispielsweise im Bereich von 1 mm³/g bis 2000 mm³/g bevorzugt im Bereich von 6 mm³/g bis 1500 mm³/g.

Die spezifische Oberfläche (BET-Oberfläche bestimmt nach DIN 66131) der gemäß Schritt B) bzw. den optionalen Schritten B2), B3) und B4) erhaltenen Katalysatoren in nicht reduziertem Zustand liegt beispielsweise bei 5 bis 150 m²/g, bevorzugt bei 5 bis 60 m²/g und besonders bevorzugt bei 5 bis 40 m²/g.

Gemäß Schritt C) werden aus den gemäß Schritt B) bzw. den optionalen Schritten B2), B3) und B4) erhaltenen Katalysator Katalysatorformkörper hergestellt.

Die Herstellung der Katalysatorformkörper kann in an sich bekannter Weise beispielsweise durch Tablettierung oder Extrudierung oder andere, dem Fachmann bekannte Methoden erfolgen.

Zur Herstellung von Katalysatorformkörpern können dem gemäß Schritt B) bzw. den optionalen Schritten B2), B3) und B4) erhaltenen Katalysator optional Hilfsmittel wie zum Beispiel Graphit, Magnesiumstearat, Zinkstearat in einer Menge von beispielsweise 0,1 bis 15 Gew.-%, bevorzugt 0,5 bis 5,0 Gew.-% bezogen auf die Menge des Katalysators bezogen auf sein Trockengewicht zugesetzt werden.

Vorzugsweise werden als Hilfsmittel Graphitpulver zugesetzt, besonders bevorzugt in einer Menge von Gew. 3,0 bis 5,0 Gew.-%, bezogen auf das Trockengewicht des Katalysators.

Bei einer Formgebung durch Tablettierung wird durch die Apparateeinstellung vorzugsweise eine Seitendruckfestigkeit von 10 bis 400 N, besonders bevorzugt von 100 bis 300 N eingestellt.

Beispielhaft kann das erfindungsgemäße Verfahren wie folgt durchgeführt werden:
Schritt A):
   Das Trägermaterial wie beispielsweise Aluminiumoxidpulver wird in Wasser suspendiert und auf eine Temperatur von 20 bis 85°C temperiert. Aus einem Vorlagebehälter wird eine wässrige Lösung mit der Konzentration von 0,1 bis 3 mol/l, bevorzugt 0,5 bis 1,5 mol/l von im Wesentlichen wasserlöslicher Kupferverbindung, bevorzugt Kupfernitrat, und eine entsprechende Menge von im Wesentlichen wasserlöslicher Zinkverbindung, bevorzugt Zinknitrat, zu dem suspendierten Aluminiumoxidpulver gepumpt. Gleichzeitig wird eine wässrige Lösung mit der Konzentration von 0, 1 bis 5 mol/l, bevorzugt 0,5 bis 2 mol/l einer Base, bevorzugt Ammoniumcarbonat, Natriumcarbonat, Natronlauge oder Mischungen derselben, zugepumpt. Man stellt die Zugabegeschwindigkeit der beiden Lösungen so ein, dass der pH-Wert bei der Temperatur, bei der die Fällung vorgenommen wird, vorzugsweise innerhalb des Bereichs von 7,5 bis 9,0 gehalten wird. Die Fällung wird bei einer möglichst konstanten Temperatur im Bereich von vorzugsweise 20 bis 85°C durchgeführt. Im Anschluss an die Fällung lässt man die entstandene Suspension bei einer Temperatur von vorzugsweise 20 bis 85°C über einen Zeitraum von 0,5 bis 3 Stunden nachrühren.
Schritte B), B2) und B3):
   Anschließend wird filtriert und mit Wasser, bevorzugt bei 15 bis 70°C, besonders bevorzugt bei 15 bis 25°C gewaschen.
   Der Filterkuchen kann vorzugsweise bei Temperaturen von 90 bis 120°C gegebenenfalls im Vakuum getrocknet. Die Trocknung kann auch gleichzeitig mit einer Sprühagglomeration, z.B. in einem Sprühtrockner, zu Partikeln mit einem weitgehend einheitlichen Durchmesser, vorzugsweise im Bereich von 10 bis 80 µm durchgeführt werden.
Schritt C):
   Aus dem getrockneten, gefällte Katalysator werden anschließend durch Tablettierung oder durch Extrudierung Katalysatorformkörper hergestellt.

Die erfindungsgemäß hergestellten Katalysatorformkörper zeichnen sich insbesondere durch eine hohe Hydrieraktivität und eine besondere mechanische und chemische Stabilität, insbesondere beim Einsatz in Festbettreaktoren mit Flüssigfahrweise aus.

Beim Einsatz in Hydrierungen wird der in den Katalysatorfortnkörpern enthaltene, nicht reduzierte Katalysator durch den Wasserstoff in seine entsprechende reduzierte Form überführt.

In einer weiteren Ausführungsform der Erfindung kann der Katalysator jedoch auch vor dem Einsatz in Hydrierungen in einem Schritt D) in die reduzierte Form überführt werden.

Das Überführen in die reduzierte Form kann beispielsweise mit Wasserstoff, Hydrazin, Methan oder anderen Kohlenwasserstoffen oder anderen Reduktionsmitteln erfolgen

Die Reduktion erfolgt vorzugsweise mit Wasserstoff oder einem Gemisch von Wasserstoff mit einem Inertgas wie beispielsweise Stickstoff, Argon oder anderen Edelgasen.

In einer bevorzugten Ausführungsform wird der Katalysatorformkörper beispielsweise zunächst unter einem Gemisch aus 95 Vol.-% Stickstoff und 5 Vol.-% Wasserstoff mit einem auf die Reduktionstemperatur gebracht, dann wird schrittweise der Wasserstoffanteil verändert, bis der gewünschte Wasserstoffanteil erreicht wird. Am Ende der Reaktion wird beispielsweise zunächst der Wasserstoffanteil wieder auf 0 Vol.-% erniedrigt, was vorzugsweise schrittweise erfolgt, anschließend wird der verwendete Stickstoff schrittweise durch Luft ersetzt.

Die Temperatur bei der Reduktion beträgt beispielsweise von 100 bis 800°C, bevorzugt 200 bis 350°C, die Reaktionszeit liegt dabei beispielsweise zwischen 15 Minuten und 24 Stunden, bevorzugt zwischen 2 und 20 Stunden.

Der Druck bei der Reduktion beträgt beispielsweise von 1 bis 300 bar, bevorzugt von 1 bis 8 bar, besonders bevorzugt bei 1 bar.

Zur Ermittlung der Seitendruckfestigkeit wird der zylindrische Katalysatorformkörper mit einer Dimension von 5mm Durchmesser und 5mm Länge auf die Seite zwischen zwei flache Stempel eingebracht. Die Stempel fahren mit einer Geschwindigkeit von 15mm/min aufeinander zu. Ein Kraftaufnehmer misst die maximale Kraft, die aufgewendet wurde, bevor die Tablette zerbricht. Sofern nicht anders angegeben, wurde die Seitendruckfestigkeit mit Hilfe eines Instron Mini 44 gemessen

In einer standardisierten Ausführungsform erfolgt die Reduktion analog zu Beispiel 8:
Die erfindungsgemäßen Katalysatorformkörper weisen nach Schritt D), der Reduktion mit Wasserstoff eine sehr hohe Seitendruckfestigkeit von mindestens 8 N, bevorzugt mindestens 12 N, besonders bevorzugt mindestens 14N und ganz besonders bevorzugt mindestens 15 N auf, die für solche Katalysatortypen bislang unerreicht ist.

Von der Erfindung sind daher auch Katalysatorformkörper enthaltend Kupfer, Zink und Aluminium umfasst, die im reduzierten Zustand eine Seitendruckfestigkeit von mindestens 8 N, bevorzugt mindestens 12 N, besonders bevorzugt mindestens 14N und ganz besonders bevorzugt mindestens 15 N aufweisen, sowie Katalysatorformkörper, die durch das erfindungsgemäße Verfahren erhältlich sind.

Für die Katalysatorformkörper gelten im Übrigen die gleichen Vorzugsbereiche, einschließlich der Vorzugsbereiche der darin enthaltenen Katalysatoren wie vorstehend für das Verfahren angegeben.

Ebenfalls von der Erfindung umfasst ist die Verwendung von Aluminium und Sauerstoff enthaltenden Trägermaterialien für die Herstellung der erfindungsgemäßen Katalysatorformkörper, wobei für die Aluminium und Sauerstoff enthaltenden Trägermaterialien die oben genannten Vorzugsbereiche in gleicher Weise gelten.

Besonders vorteilhaft lassen sich die erfindungsgemäßen Katalysatorformkörper in einem Verfahren zur Herstellung von Alkoholen durch Hydrierung von Carbonylverbindungen einsetzen. Unter Carbonylverbindungen sind organische Verbindungen zu verstehen, die zumindest eine Carbonyl-Funktion aufweisen.

Beispiele für Carbonylverbindungen sind Ketone, Aldehyde, Carbonsäuren und Carbonsäureester, wobei Carbonsäuren und Carbonsäureester bevorzugt sind.

Bevorzugte Carbonsäuren und Carbonsäureester sind α,ω-Dicarbonsäuren oder deren Ester, besonders bevorzugt sind Adipinsäure und deren Ester, wie insbesondere deren Methyl-, Ethylester sowie Ester der Adipinsäure mit Hexan-1,6-diol, die sogenannten Adipoladipinate.

Daher umfasst die Erfindung weiterhin die Verwendung der erfindungsgemäßen Katalysatorformkörper in einem Verfahren zur Herstellung von Alkoholen durch Hydrierung von Carbonylverbindungen, sowie das Verfahren selbst.

Das erfindungsgemäße Verfahren wird vorzugsweise wird bei einer Temperatur von 100 bis 350°C, besonders bevorzugt bei 150 bis 300°C und besonders bevorzugt bei 200 bis 280°C durchgeführt.

Der Druck, beim dem das erfindungsgemäße Verfahren durchgeführt wird, beträgt bevorzugt 50 bis 400 bar, besonders bevorzugt 100 bis 300 bar.

Es kann in einem Reaktor oder auch in mehreren hintereinandergeschalteten Reaktoren gearbeitet werden.

Das erfindungsgemäße Verfahren kann sowohl ohne als auch mit Zusatz von Lösungsmitteln, z.B. Alkoholen, durchgeführt werden.

Bei der Umsetzung von Carbonsäuren mit Wasserstoff ist es vorteilhaft, die Hydrierung in einem Alkohol als Lösungsmittel durchzuführen.

Geeignete Alkohole sind beispielsweise Methanol, Ethanol, Isopropanol, n-Propanol, Butandiol oder Hexan-1,6-diol. Bevorzugt setzt man den Alkohol als Lösungsmittel ein, der durch die Hydrierung der jeweiligen Carbonsäure entsteht.

Bevorzugte Ausführungsformen des Katalysators bzw. der Katalysatorformkörper entsprechen denen, die bereits bei der Beschreibung des Verfahrens angeführt wurden.

Im Folgenden wird die Erfindung anhand von Beispielen weiter erläutert. Die Beispiele stellen einzelne Ausführungsformen der Erfindung dar, die Erfindung ist jedoch nicht auf die Beispiele beschränkt.

### Beispiele

### Beispiel 1: Herstellung einer Metallsalzlösung

In einem beheizbaren 401 Doppelmantelgefäß aus Glas wurden 6913,9g HNO₃ (71,4 mol, 65%ig) mit 27.000 g deionisiertem Wasser gemischt. Die Lösung wurde unter Rühren auf 70°C erhitzt. Es wurden dann bei 70 bis 74°C 2196,9 g Kupfer(II)oxid (27,21mol, 98%ig) und 696,5g Zinkoxid (8,49 mol, 98%ig) als Mischung zugeben. Anschließend wurde 30 Minuten bei 70°C nachgerührt und eine blaue Lösung mit einem pH-Wert von 0,9 erhalten. Die Lösung wurde auf Raumtemperatur abgekühlt, filtriert und in einem Kunststoffgefäß zur Verwendung in den weiteren Beispielen gelagert.

### Beispiel 2: Herstellung von mechanisch stabilem Hydrierkatalysator auf Basis von Aluminiumoxid HiQ 7214F der Firma BASF SE

18.198 g der Metallsalzlösung aus Beispiel 1 wurde in einem 151 Doppelmantelgefäß aus Glas (der Doppelmantel wurde über eine Heizflüssigkeit mittels eines Thermostaten beheizt) vorgelegt und auf 70°C Innentemperatur (Außentemperatur 77°C) aufgeheizt und dann bei dieser Temperatur gehalten. Ebenso wurde in einem zweiten, 101 Doppelmantelgefäß 14.000 g einer 13,05 Gew.-% igen wässrigen Natriumcarbonatlösung vorgelegt und auf 70°C Innentemperatur (Außentemperatur 77°C) aufgeheizt.

Der 401-Doppelmantelgefäß-Fällreaktor wurde ausgestattet mit einer pH - Elektrode, einem Thermoelement zur Innentemperaturmessung, einem Kreuzblattrührer (Blattlänge etwa 7cm, Blatthöhe etwa 2 cm, 4 Blätter), sowie zwei gegenüberliegenden Dosieröffnungen. Die Rührerdrehzahl betrug 150 U/min. Alle von der Lösung benetzten Einbauten waren aus Glas oder Kunststoff.

Der Fällreaktor wurde mit 4.000 g deionisiertem Wasser und 61,5g Aluminiumoxid der Firma BASF (HiQ 7214F, 0,603 mol) befüllt und auf 70°C Innentemperatur (Außentemperatur 76°C) aufgeheizt.

Das Aluminiumoxid HiQ 7214F der Firma BASF SE wies eine BET-Oberfläche von 152 m²/g, eine Helium-Dichte von 3,403 g/cm³, eine Rohdichte von 0,741 g/cm3, ein Porenvolumen von 1,055 cm³/g und eine Porosität von 78,2 % auf. Relativ häufigste Durchmesser wurden für 9,7 µm und 11 nm gemessen.

Nach Erreichen der Innentemperatur im Reaktor wurde durch Eindosieren der Natriumcarbonatlösung auf pH 8,3 gestellt und dann beide Lösungen mit einer Temperatur von ebenfalls 70°C gleichzeitig durch die gegenüberliegenden Reaktoröffnungen aus den Vorlagegefäßen zudosiert. Dabei wurde ein pH-Wert von 7,9 bis 8,1 erreicht und während der Dosierung gehalten. Die Dosierzeit betrug insgesamt etwa 6 Stunden.

Anschließend wurde 2 Stunden bei 70°C nachgerührt und der ausgefallene Feststoff abgesaugt und mehrmals mit jeweils 8 Liter deionisiertem Wasser dekantierend gewaschen. Der ausgefallene Feststoff wurde im Trockenschrank bei 110°C über Nacht getrocknet.

Es wurden insgesamt drei Fällungen durchgeführt und dabei folgende Ausbeuten und physikalische Parameter ermittelt:

| | | |
|---|---|---|
| Fällung 1: 2016g | 0,5% Wasser | Schüttgewicht 945g/l |
| Füllung 2: 2014g | 0,4% Wasser | Schüttgewicht 1028g/l |
| Fällung 3: 2013g | 0,34% Wasser | Schüttgewicht 1090g/l |

Die gemittelte Analyse der chemischen Zusammensetzung ergab für die Katalysatoren einen Gehalt von 41,09 Gew.-% Kupfer, 12,65 Gew.-% Zink und 1,54 Gew.-% Aluminium (jeweils berechnet auf das Metall).

Zu diesem Katalysator wurden 3,0 Gew.-% Graphit Typ: T44 (Hersteller Lonza, Schweiz) zugemischt und die resultierende Mischung anschließend zu Tabletten mit einem Durchmesser und einer Höhe von jeweils 5 mm dergestalt verformt, so dass die Tabletten am Ende des Verformungsvorgangs eine Seitendruckfestigkeit von im Mittel 140 N besaßen.

Das Schüttgewicht der Tabletten betrug 1,9 kg/l Die BET-Oberfläche (bei 77K mit Stickstoff nach DIN ISO 9277) lag bei 11,4 m²/g.

Die Kristallitgröße des im Katalysator enthaltenen Malachits lag errechnet am Reflex [0/2/0] bei 48,7 nm.

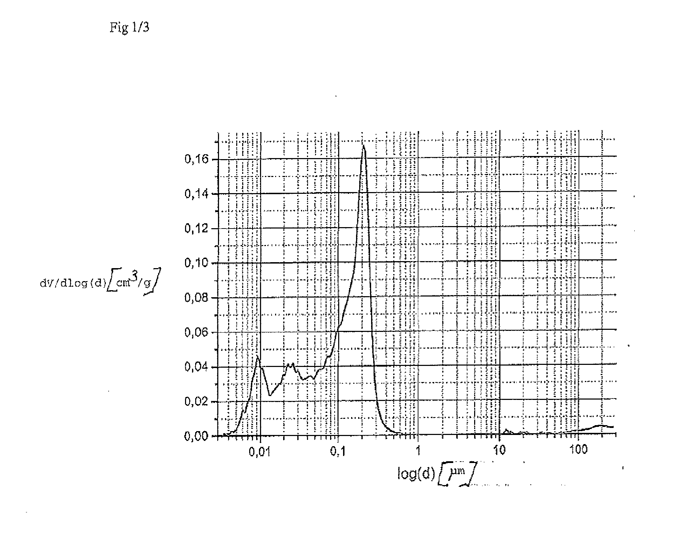

Die Porengrößenverteilung des Katalysators gemäß Beispiel 2 ist in Fig. 1 wiedergegeben.

### Beispiel 3: Herstellung von mechanisch stabilem Hydrierkatalysator auf Basis von Aluminiumoxid HiQ 7214F der Firma BASF SE nach Aufmahlen der Fällung

Völlig analog zu Beispiel 1 wurden insgesamt drei Fällungen durchgeführt und dabei folgende Ausbeuten und physikalische Parameter ermittelt:

| | | |
|---|---|---|
| Fällung 1: 1994g; | 0,34 Gew.-% | Wasser Schüttgewicht 968g/l |
| Fällung 2: 2005g; | 0,38 Gew.-% | Wasser Schüttgewicht 970g/l |
| Fällung 3: 2001g; | 0,18 Gew.-% | Wasser Schüttgewicht 944g/l |

Die drei Fällungen wurden vereinigt und in einer Kugelmühle (Fa. Retsch, Deutschland) 15 min bei 250 U/min gemahlen und homogenisiert. Das Schüttgewicht des so erhaltenen Materials lag bei 1.598 g/1.

Die gemittelte Analyse der chemischen Zusammensetzung ergab für die Katalysatoren einen Gehalt von 42,28 Gew.-% Kupfer, 12,88 Gew.-% Zink und 1,60 Gew.-% Aluminium (jeweils berechnet auf das Metall).

Zu diesem Katalysator wurden 3,0 Gew.-% Graphit Typ: T44 (Hersteller Lonza, Schweiz) zugemischt und die resultierende Mischung anschließend zu Tabletten mit einem Durchmesser und einer Höhe von jeweils 5 mm dergestalt verformt, so dass die Tabletten am Ende des Verformungsvorgangs eine Seitendruckfestigkeit von im Mittel 215 N besaßen.

Das Schüttgewicht der Tabletten betrug 1.922 g/1.. Die BET-Oberfläche (bei 77K mit Stickstoff nach DIN ISO 9277) lag bei 12,5 m²/g.

Die Kristallitgröße des im Katalysator enthaltenen Malachits lag errechnet am Reflex [0/2/0] bei 49,0 nm.

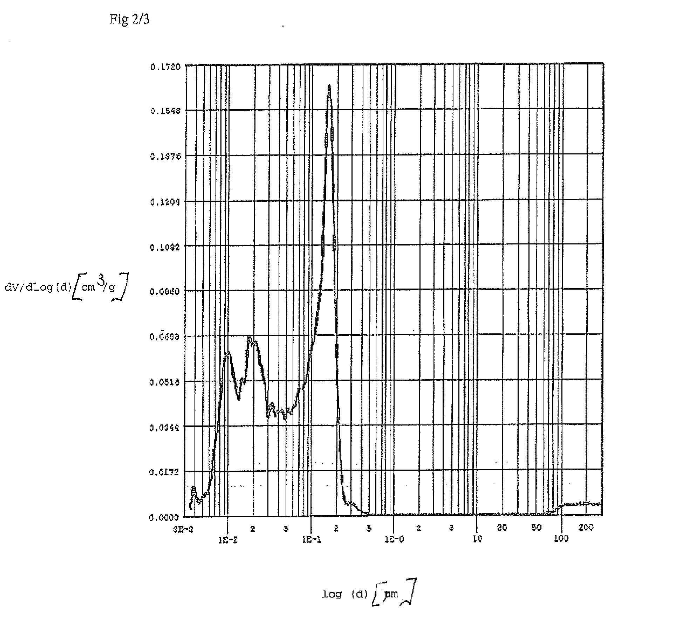

Die Porengrößenverteilung des Katalysators gemäß Beispiel 3 ist in Fig. 2 wiedergegeben.

### Beispiel 4: Herstellung von mechanisch stabilem Hydrierkatalysator auf Basis von Böhmit (Pural NF der Firma Sasol, Süd-Afrika)

Analog zu Beispiel 1 mit dem Unterschied, dass der Fällreaktor mit 4.000 g deionisiertem Wasser und 79,9 g Böhmit, einem orthorhomisch kristallisierenden basischen Aluminiumoxid der ungefähren Zusammensetzung γ-AIO(OH) der Firma der Firma Sasol (Plural NF) befüllt (pH 5,6) wurde, wurde eine Fällung durchgeführt, wobei während der 6- stündigen Dosierung ein pH-Wert von 7,7 bis 8,3 gehalten wurde.

Der eingesetzte Böhmit wies eine BET-Oberfläche von 146 m²/g, eine Helium-Dichte von 2,901 g/cm³, eine Rohdichte von 0,972 g/cm3, ein Porenvolumen von 0,684 cm³/g und eine Porosität von 66,5 % auf. Relativ häufigste Durchmesser wurden für 1,5 µm und 11 nm gemessen.

Es wurde nach Absaugen, mehrmaligem dekantierendem Waschen mit jeweils 4 Liter deionisiertem Wasser und Trocknen des ausgefallenen Feststoffs im Trockenschrank bei 110°C über Nacht folgende Ausbeuten und physikalische Parameter ermittelt:
Ausbeute: 2015g; 0,98 Gew.-% Wasser.

Die gemittelte Analyse der chemischen Zusammensetzung ergab für die Katalysatoren einen Gehalt von 42,48 Gew.-% Kupfer, 13,10 Gew.-% Zink und 1,57 Gew.-% Aluminium (jeweils berechnet auf das Metall).

Zu diesem Katalysator wurden 3,0 Gew.-% Graphit Typ: T44 (Hersteller Lonza, Schweiz) zugemischt und die resultierende Mischung anschließend zu Tabletten mit einem Durchmesser und einer Höhe von jeweils 5 mm dergestalt verformt, so dass die Tabletten am Ende des Verformungsvorgangs eine Seitendruckfestigkeit von im Mittel 220 N besaßen.

Die BET-Oberfläche (bei 77K mit Stickstoff nach DIN ISO 9277) lag bei 38,0 m²/g.

Die Kristallitgröße des im Katalysator enthaltenen Malachits lag errechnet am Reflex [0/2/0] bei 9,7 nm.

### Beispiel 5: Herstellung von mechanisch stabilem Hydrierkatalysator auf Basis eines α-Alumiuiumoxides (Typ SA5396 der Firma Saint-Gobain, Frankreich)

Analog zu Beispiel 1 mit dem Unterschied, dass der Fällreaktor mit 4.000 g deionisiertem Wasser und 61,5g α-Aluminiumoxid Typ SA5396 der Firma Saint-Gobain befüllt (pH 8,2) wurde, wurde eine Fällung durchgeführt, wobei während der 6- stündigen Dosierung ein pH-Wert von 7,7 bis 8,3 gehalten wurde.

Das eingesetzte α-Aluminiumoxid wies eine BET-Oberfläche von 0,3 m²/g auf.

Der ausgefallene Feststoff wurde in eine Rahmenfilterpresse gepumpt mit insgesamt 52 1 entionisiertem Wasser nachgewaschen, bis das Filtrat nitratfrei war. Der Filterkuchen wurde dann zunächst trockengeblasen, dann in wenig Wasser resuspendiert und anschließend sprühgetrocknet. Es wurden folgende Ausbeuten und physikalische Parameter ermittelt:
Ausbeute: 1.915 g mit 32,65 Gew.-% Wasser

Die gemittelte Analyse der chemischen Zusammensetzung ergab für die Katalysatoren einen Gehalt von 42,22 Gew.-% Kupfer, 13,11 Gew.-% Zink und 1,16 Gew.-% Aluminium (jeweils berechnet auf das Metall).

Zu diesem Katalysator wurden 3,0 Gew.-% Graphit Typ: T44 (Hersteller Lonza, Schweiz) zugemischt und die resultierende Mischung anschließend zu Tabletten mit einem Durchmesser und einer Höhe von jeweils 5 mm dergestalt verformt, so dass die Tabletten am Ende des Verformungsvorgangs eine Seitendruckfestigkeit von im Mittel 117 N besaßen.

Die BET-Oberfläche (bei 77K mit Stickstoff nach DIN ISO 9277) lag bei 15,8 m²/g.

Die Kristallitgröße des im Katalysator enthaltenen Malachits lag errechnet am Reflex [0/2/0] bei 26,3 nm.

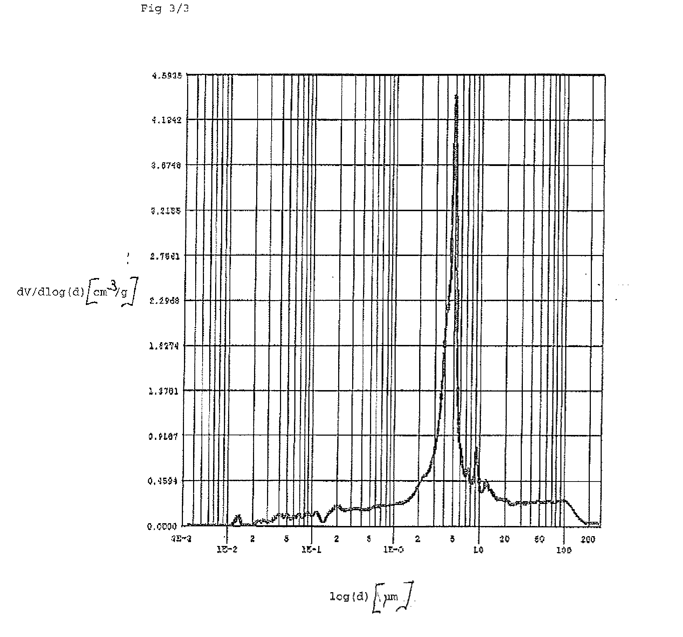

Die Porengrößenverteilung des Katalysators gemäß Beispiel 5 ist in Fig. 3 wiedergegeben.

### Beispiel 6: Herstellung von mechanisch stabilem Hydrierkatalysator auf Basis eines nitrathaitigen Aluminiumoxidhydroxides (Chargen-Nr. 2009850063 der Firma Saint-Gobain, Frankreich)

Analog zu Beispiel 1 mit dem Unterschied, dass der Fällreaktor mit 4.000 g deionisiertem Wasser und 61,5g des Aluminiumoxidhydroxides (Nr. 2009850063) befüllt (pH 3,0) wurde, wurde eine Fällung durchgeführt, wobei während der 6- stündigen Dosierung ein pH-Wert von 7,7 bis 8,3 gehalten wurde.

Das eingesetzte γ-Aluminiumoxid wies eine BET-Oberfläche von 101 m²/g auf.

Es wurde nach Absaugen, mehrmaligem dekantierendem Waschen mit jeweils 4 Liter deionisiertem Wasser und Trocknen des ausgefallenen Feststoffs im Trockenschrank bei 110°C über Nacht folgende Ausbeuten und physikalische Parameter ermittelt:
Ausbeute: 1.864 g mit 3,04 Gew.-% Wasser

Die gemittelte Analyse der chemischen Zusammensetzung ergab für die Katalysatoren einen Gehalt von 42,33 Gew.-% Kupfer, 13,42 Gew.-% Zink und 1,32 Gew.-% Aluminium (jeweils berechnet auf das Metall).

Zu diesem Katalysator wurden 3,0 Gew.-% Graphit Typ: T44 (Hersteller Lonza, Schweiz) zugemischt und die resultierende Mischung anschließend zu Tabletten mit einem Durchmesser und einer Höhe von jeweils 5 mm dergestalt verformt, so dass die Tabletten am Ende des Verformungsvorgangs eine Seitendruckfestigkeit von im Mittel 180 N besaßen.

Die erhaltenen Tabletten wurden auf einem Frewitt-Sieb wieder aufgemahlen und erneut wie oben tablettiert.

Die BET-Oberfläche (bei 77K mit Stickstoff nach DIN ISO 9277) lag bei 39,6 m²/g.

Die Kristallitgröße des im Katalysator enthaltenen Malachits lag errechnet am Reflex [0/2/0] bei 9,9 nm.

### Beispiel 7 (zum Vergleich)

Die Herstellung des Hydrierkatalysators erfolgte nach Beispiel 7 aus DE 102 074 43 A.

### Beispiel 8: Reduktion der Katalysatflrfarmkörper

Bei allen Katalysatoren wurde die Seitendruckfestigkeit nach einer Reduktion im Wasserstoffstrom unter Normaldruck gemessen, um damit eine von einer einzelnen Hydrierreaktion unabhängigen Aussage der mechanischen Stabilität der Katalysatorformkörper (Katalysatortabletten) während einer solchen Reaktion zu erhalten. Hierzu wurden jeweils etwa 100g der jeweiligen Katalysatorformkörper in einem Quarzgefäß mit folgendem Programm reduziert. Die Probe wurde am Ende des Programms ausgebaut und die Seitendruckfestigkeit gemessen. In Schritt sieben wird langsam auf 25°C abgekühlt, die höheren Temperaturen in den Schritten 9 und 10 sind auf Exothermieeffekte zurückzuführen.

| Schritt | Dauer [min] | Probentemperatur [°C] | 5% Wasserstoff in Stickstoff [L/h] | Stickstoff [L/h] | Wasserstoff [L/h] | Luft [L/h] |
|---|---|---|---|---|---|---|
| 1 | 180 | auf 250 | 10 | 0 | 0 | 0 |
| 2 | 45 | 250 | 0 | 18 | 2 | 0 |
| 3 | 30 | 250 | 0 | 16 | 4 | 0 |
| 4 | 45 | 250 | 0 | 14 | 6 | 0 |
| 5 | 45 | 250 | 0 | 12 | 8 | 0 |
| 6 | 48 | 250 | 0 | 10 | 10 | 0 |
| 7 | 600 | auf 25 | 0 | 10 | 10 | 0 |
| 8 | 15 | 35 | 0 | 20 | 0 | 1,3 |
| 9 | 30 | 35 | 0 | 20 | 0 | 3,4 |
| 10 | 60 | 25 | 0 | 20 | 0 | 5,1 |
| 11 | 30 | 25 | 0 | 15 | 0 | 10 |
| 12 | 30 | 25 | 0 | 10 | 0 | 15 |
| 13 | 30 | 25 | 0 | 5 | 0 | 20 |
| 14 | 45 | 25 | 0 | 2 | 0 | 20 |

Der gemessene Gewichtsverlust lag für alle Katalysatoren der Beispiele 2 bis 7 bei etwa 36%.

Die gemessene, gemittelte Seitendruckfestigkeit ist in Tabelle 1 angegeben. Bei den angegebenen Seitendruckfestigkeiten handelt es sich um arithmetische Mittelwerte aus der Messung von 10 Tabletten. Bruch der Tabletten wurde für die erfindungsgemäßen Katalysatoren nicht beobachtet.

**Tabelle 2: Seitendruckfestigkeiten der Katalysatorformkörper**

| Katalysator aus Bsp. | Mittlere Seitendruckfestigkeit im nicht-reduzierten Zustand [N] | Mittlere Seitendruckfestigkeit im reduzierten Zustand [N] |
|---|---|---|
| 2 | 140 | 15 |
| 3 | 215 | 15 |
| 4 | 220 | nicht gemessen |
| 5 | 117 | nicht gemessen |
| 6 | 180 | 19 |
| 7 (Vergleich) | 158 | *) |

| | | |
|---|---|---|
| *) Seitendruckfestigkeiten nicht messbar, da im wesentlichen Pulver oder Bruch der Katalysatortabletten vorlag. | | |

### Beispiel 9: Einsatz der Katalysatorformkörper in Hydrierungen

Um zu prüfen, ob die Katalysatorformkörper des Beispiels 2 auch in Hydrierreaktionen eingesetzt werden können, wurde der Katalysatorformkörper in Analogie zu Beispiel 8 aus DE 102 074 43 A, zur Hydrierung von Adipoladipinat getestet.

Dazu wurde ein senkrecht stehendes, wärmeisoliertes Hochdruckrohr aus nichtrostendem, säurefestem Material von 45 mm Durchmesser und 1 m Länge, das vorher mit Stickstoff sauerstofffrei gespült wurde, mit 1,4 1 der Katalysatoren nach Beispiel 2 gefüllt. Zur Aktivierung des Katalysators wird zunächst 6 Stunden lang bei 200°C ein Stickstoffstrom (5 Nm³/h) durch das Katalysatorbett geleitet. Nun erfolgt die Reduktion des Katalysators, indem bei einem Stickstoffdruck von 200 bar und einer Temperatur zwischen 180°C und 200°C langsam Wasserstoff zugemischt wird, wobei der Anfangsgehalt 10 bis 15 Vol.-% nicht übersteigen sollte. Über den Zeitraum von 24 h wird der Stickstoffanteil mehr und mehr vermindert, bis letztlich reiner Wasserstoff durch den Reaktor strömt. Die Reaktion ist beendet, wenn kein Reaktionswasser mehr gebildet wird.

Nach der Aktivierung des Katalysators wird der Wasserstoffdruck auf 300 bar erhöht und ein Volumenstrom von 5 Nm³/h eingestellt. Nun wird Hexandiol-1,6-adipat durch den Reaktor gefördert, das durch Veresterung von Adipinsäure mit 1,6-Hexandiol im Verhältnis 1:1,1 erhalten wurde (vgl. EP-A 0 721 928). Die Zulaufmenge wurde auf 200g/h eingestellt, die Temperatur auf 240°C.

Das aus dem Reaktionsrohr austretende Reaktionsgemisch wird in einem zweiten Wärmeaustauscher (Wasserkühler) bei 300 bar Wasserstoffdruck auf kleiner 60°C gekühlt und in einem Gasabscheider von überschüssigem Wasserstoff, der wieder in das Hydriersystem zurückgeführt wird, getrennt. Nach weiterer Abkühlung auf eine Temperatur kleiner 30°C und Entspannung auf Normaldruck wird das Reaktionsprodukt gaschromatographisch untersucht. Die Versuchsdauer betrug 60 Stunden, der Katalysatoren war zu diesem Zeitpunkt noch unverändert wirksam.

Die Rohausbeute an 1,6-Hexandiol ist in Tabelle 2 angegeben.

Von dem Katalysatorformkörper wurde nach Ende des Versuchs die Seitendruckfestigkeit bestimmt, sie betrugt 8 N.

**Tabelle 2: Ergebnisse des Hydrierversuches**

| Beispiel | Katalysator aus Beispiel | Rohausbeute 1,6-Hexandiol |
|---|---|---|
| 9 | 2 | 98,6 % |

Die Beispiele belegen, dass die erfindungsgemäßen Katalysatorformkörper bei sehr hoher Hydrieraktivität eine im Vergleich zum Stand der Technik überlegene mechanische Stabilität aufweisen.

## Patentansprüche

1. Verfahren zur Herstellung von Kupfer, Zink und Aluminium enthaltenden Katalysatoren umfassend zumindest folgende Schritte:
A) Herstellung eines Katalysators durch
• Co-Fällung zumindest einer im Wesentlichen wasserlöslichen Kupferverbindung, zumindest einer im Wesentlichen wasserlöslichen Zinkverbindung und zumindest einer im Wesentlichen wasserlöslichen Aluminiumverbindung und/oder
• Auffällung zumindest einer im Wesentlichen wasserlöslichen Kupferverbindung und zumindest einer im Wesentlichen wasserlöslichen Zinkverbindung auf zumindest ein Aluminium und Sauerstoff enthaltendes Trägermaterial
in einem wässrigen Reaktionsmedium
B) Abtrennung des gemäß Schritt A) erhaltenen, gefällten Katalysators vom wässrigen Reaktionsmedium
C) Herstellung von Katalysatorformkörpern enthaltend den gemäß Schritt B) erhaltenen, gefällten Katalysator,
wobei das Verfahren weiterhin **dadurch gekennzeichnet ist, dass** es keine Kalzinierung des gefällten Katalysators oder der Katalysatorformkörper umfasst.

2. Verfahren nach Anspruch 1, **dadurch gekennzeichnet, dass** als im Wesentlichen wasserlösliche Kupferverbindungen Kupfer(II)-nitrat, Kupfer(II)sulfat, Kupfer(II)chlorid, Kupfer(II)bromid, Kupfer(I)acetat, Kupfer(II)acetat, oder deren Hydrate oder Mischungen der vorgenannten Verbindungen verwendet werden.

3. Verfahren nach Anspruch 1 oder 2, **dadurch gekennzeichnet, dass** als im Wesentlichen wasserlösliche Zinkverbindungen Zinknitrat, Zinksulfat, Zinkchlorid, Zinkbromid, Zinkacetat, Zinkcitrat oder deren Hydrate oder Mischungen der vorgenannten Verbindungen verwendet werden.

4. Verfahren nach einem der Ansprüche 1 bis 3, **dadurch gekennzeichnet, dass** in Schritt A) eine Auffällung auf zumindest ein Aluminium und Sauerstoff enthaltendes Trägermaterial vorgenommen wird, wobei das Trägermaterial mehr als 90 Gew.-% an Aluminum und Sauerstoff enthält.

5. Verfahren nach Anspruch 4, **dadurch gekennzeichnet, dass** als Trägermaterial γ-Aluminiumoxide, α-Aluminumoxide, β-Aluminumoxide, Böhmit, Tonerden, Kaolin, Bauxit, Gibbsite, Aluminiumoxidhydroxide oder Aluminiunihydroxide verwendet werden.

6. Verfahren nach einem der Ansprüche 4 und 5, **dadurch gekennzeichnet, dass** als Trägermaterial solche verwendet werden, die eine Partikelgröße von 1 bis 100 µm aufweisen.

7. Verfahren nach einem der Ansprüche 4 bis 6, **dadurch gekennzeichnet, dass** als Trägermaterial solche verwendet werden, die eine spezifische Oberfläche von 0, 1 bis 400 m²/g aufweisen.

8. Verfahren nach einem der Ansprüche 4 bis 7, **dadurch gekennzeichnet, dass** als Trägermaterial solche verwendet werden, die ein Porenvolumen von 0,1 bis 1,5 ml/g, aufweisen.

9. Verfahren nach einem der Ansprüche 1 bis 8, **dadurch gekennzeichnet, dass** bei der Auf- oder Co-Fällung der pH-Wert des wässrigen Reaktionsmediums durch Zugabe von Basen auf 6,0 bis 12,0 eingestellt wird.

10. Verfahren nach Anspruch 9, **dadurch gekennzeichnet, dass** als Basen Alkalimetallcarbonate verwendet werden.

11. Verfahren nach Anspruch 10, **dadurch gekennzeichnet, dass** der durch Verwendung von Alkalimetallcarbonaten gefällte Malachit eine durchschnittliche Kristallitgröße von 1 bis 80 nm aufweist.

12. Verfahren nach einem der Ansprüche 1 bis 11, **dadurch gekennzeichnet, dass** es zusätzlich einen Schritt B2) und/oder einen Schritt B3) und/oder einen Schritt B4) umfasst, wobei
in Schritt B2) der abgetrennte gefällte Katalysator gewaschen wird
in Schritt B3) der abgetrennte gefällte Katalysator getrocknet wird und
in Schritt B4) der abgetrennte, gefällte Katalysator gemahlen wird.

13. Verfahren nach einem der Ansprüche 1 bis 12, **dadurch gekennzeichnet, dass** zur Herstellung der Katalysatorformkörper in Schritt C) Hilfsmittel in einer Menge von 0,1 bis 15 Gew.-% bezogen auf das Trockengewicht des Katalysators zugesetzt werden.

14. Verfahren nach einem der Ansprüche 1 bis 13, **dadurch gekennzeichnet, dass** es zusätzlich einen Schritt D) aufweist, in dem der Katalysator bzw. der Katalysatorformkörper in die reduzierte Form überführt wird.

15. Katalysatorformkörper erhältlich nach einem Verfahren gemäß einem der Ansprüche 1 bis 14.

16. Katalysatorformkörper enthaltend Kupfer, Zink und Aluminium, die im reduzierten Zustand eine Seitendruckfestigkeit von mindestens 8 N aufweisen.

17. Katalysatorformkörper nach Anspruch 15 oder 16, **dadurch gekennzeichnet, dass** der enthaltene Katalysator in nicht-reduziertem Zustand bezogen auf sein Trockengewicht 2 bis 60 Gew.-% Kupfer (bezogen auf das Metall), 2 bis 25 Gew.-% an Zink (bezogen auf das Metall) und 0,1 bis 10 Gew.-% an Aluminium enthält.

18. Katalysatorformkörper nach einem der Ansprüche 15 bis 17, **dadurch gekennzeichnet, dass** der enthaltene Katalysator eine oder mehrere weitere Metallverbindungen enthält die ausgewählt sind aus den Verbindungen der Seltenerdmetalle, Alkalimetalle und Erdalkalimetalle, sowie den Verbindungen von Zirkon, Titan, Cobalt, Molybdän, Vanadium, Wolfram, Eisen, Nickel, Mangan oder Rhenium oder Mischungen der vorgenannten Metalle oder Verbindungen.

19. Verfahren zur Herstellung von Alkoholen durch Hydrierung von Carbonylverbindungen, **dadurch gekennzeichnet dass** es in Gegenwart von Katalysatorformkörpern nach einem der Ansprüche 15 bis 18 durchgeführt wird.

20. Verwendung von Aluminium und Sauerstoff enthaltenden Trägermaterialien für die Herstellung von Katalysatorformkörpern nach einem der Ansprüche 15 bis 18.
